# FASCICULE DE BREVET EUROPEEN

(11) **EP 2 556 133 B1**
(45) Date de publication et mention de la délivrance du brevet: **07.06.2017**
(21) Numéro de dépôt: 11719667.5
(22) Date de dépôt: 07.04.2011
(51) Int. Cl.: C10G 27/04, C10G 27/12, C10G 27/14, C12N 9/08, C12P 5/00

(54) **PROCEDE DE PURIFICATION DES EXTRAITS AROMATIQUES CONTENANT DES COMPOSES POLYCYCLIQUES AROMATIQUES**
VERFAHREN ZUR AUFREINIGUNG VON AROMATISCHEN EXTRAKTEN MIT AROMATISCHEN POLYCYCLISCHEN VERBINDUNGEN
PROCESS FOR PURIFYING AROMATIC EXTRACTS CONTAINING AROMATIC POLYCYCLIC COMPOUNDS

(30) Priorité: 09.04.2010 FR 1052733
(43) Date de publication de la demande: 13.02.2013
(73) Titulaire: TOTAL MARKETING SERVICES, 92800 Puteaux (FR)
(72) Inventeur: HEDHLI, Lotfi, F-78590 Noisy Le Roi (FR); DJELASSI, Samuel, F-10600 La Chapelle Saint-Luc (FR); PULVIN, Sylviane, F-60200 Compiegne (FR); THOMAS, Daniel, F-60150 Villers Sur Coudun (FR)
(74) Mandataire: Hirsch & Associés
(86) Numéro de dépôt international: PCT/IB2011/051503
(87) Numéro de publication internationale: WO 2011/125042

(56) Documents cités:
- EP-A1- 0 417 980
- EP-A2- 0 839 891
- WO-A2-2007/006925
- US-B1- 6 461 859

## Description

La présente invention concerne un procédé de purification des extraits aromatiques issus du raffinage de pétrole brut contenant des hydrocarbures aromatiques polynucléaires hautement toxiques ou HAP (ou PAH en anglais, Polycyclic Aromatic Hydrocarbons). Et qui sont utilisés comme plastifiants dans les industries du caoutchouc industriel et du pneumatique.

Les extraits aromatiques sont des produits secondaires du procédé d'obtention des lubrifiants dans les processus de raffinage des pétroles bruts, notamment à partir des produits de la distillation sous vide des résidus atmosphériques. Ils résultent d'une simple ou d'une double extraction du raffinat valorisable dans les lubrifiants, au moyen d'un solvant polaire, par exemple du furfural, du phénol ou de N-méthylpyrrolidone ou encore de l'hydrotraitement des ces distillats et d'une étape de déparaffinage. Ces extraits sont référencés à l'ETRMA (european tire and rubber manufacturers association) et répondent à la définition de « combinaison complexe d'hydrocarbures de C20 to C50 obtenus par (1) extraction au solvant des distillats pétroliers lourds or (2) traitement des distillats pétroliers lourds à l'hydrogène en présence d'un catalyseur ; suivi par un déparaffinage au solvant ». Les DAE ou distillate aromatic extract (extrait aromatique de distillat) étaient jusqu'à présent les seuls à répondre entièrement au cahier des charges de l'industrie du pneumatique et de ce fait étaient incontournables. D'autres extraits trouvent peu à peu une place dans les formulations pneumatiques à cause de l'interdiction des DAE, tels que le MES ou Mild Extract Solvant, ou encore de TDAE ou treated aromatic extract (extrait distillé aromatique en français). Certains extraits sont issus du désasphaltage des résidus sous vide suivi d'une simple ou d'une double extraction et sont appelés respectivement RAE ou Residual aromatic extract (extrait aromatique résiduel en français) et TRAE ou treated residual aromatic extract (extrait aromatique résiduel traité par double extraction en français).

L'extrait aromatique contient typiquement de 10 à 50% en poids d'aromatiques polycycliques ou PCA en anglais (polycyclic aromatic hydrocarbons) 25 à 60% en poids de composés paraffiniques, de 45 à 20% de composés naphténiques. Les extraits aromatiques sont très utilisés dans l'industrie du pneumatique et du caoutchouc industriel car ils favorisent la réduction des pertes d'énergie ce qui permet d'augmenter la production de ces pneumatiques ou caoutchoucs. En outre, ils améliorent les propriétés mécaniques et les performances à basse température des caoutchoucs dans lesquels ils sont incorporés.

Pour rappel, les composés PCA sont des molécules composées d'au moins deux cycles de 5 à 6 atomes de carbone dont au moins deux atomes de carbone sont mis en commun et au moins l'un des cycles est aromatique. Ces composés PCA diffèrent par le nombre et l'agencement des cycles aromatiques ainsi que par les substitutions de chaînes alkyles liées aux noyaux aromatiques. Selon le nombre de cycles, les PCA sont classés en composés PCA légers (jusqu'à trois cycles) ou PCA lourds (au-delà de trois cycles), et ont des caractéristiques physico-chimiques et toxicologiques très différentes. Certains de ces composés aromatiques polycycliques PCA comprennent au moins un hétérocycle avec au moins un atome de soufre, d'azote et/ou d'oxygène. Parmi ces composés PCA, certains appelés HAP ou hydrocarbures aromatiques polynucléaires sont classés dans la liste des polluants majeurs dangereux pour la santé publique du fait de leur caractère toxique. Autre particularité, les HAP ne comprennent pas dans leur structure chimique d'hétérocycles contrairement aux PCA. Ces composés HAP sont des molécules biologiquement actives qui peuvent être absorbées par des organismes vivants. Parmi ces composés HAP, le Benzo(a)pyrène (B(a)P), le chrysène et le benzo(b)fluoranthène sont connus pour être particulièrement toxiques. Selon la structure chimique des métabolites avec lesquels ils sont mis en contact et leur caractère mutagène, ces composés HAP toxiques peuvent entraîner une diminution de la réponse du système immunitaire augmentant ainsi les risques d'infection, voire être cancérigènes.

Pour obliger les producteurs d'extraits aromatiques à réduire la teneur en HAP toxiques présents dans les extraits aromatiques susceptibles d'avoir une influence négative sur l'environnement, les instances européennes ont publié une nouvelle directive en 2005 (DIRECTIVE 2005/69/CE) qui fixe les limites de la concentration en benzo(a)pyrène à 1ppm et celle de la totalité des HAP toxiques cités dans la directive à 10ppm, ce qui correspond à des concentrations environ dix fois inférieures aux concentrations actuelles.

Face à cette directive, les producteurs d'extraits aromatiques ont tenté de diminuer la teneur en HAP toxiques dans les extraits aromatiques ou DAE par différents procédés.

Un des premiers procédés testés a été d'effectuer une seconde extraction au solvant de l'extrait aromatique comme il est décrit dans 1 brevet EP417980 typiquement avec du furfural. Le produit fabriqué correspond au TDAE. Le procédé est très coûteux en solvant, les rendements ne sont pas toujours satisfaisants, et le TDAE a une composition chimique différente des DAE et donc des propriétés physico-chimiques très différentes.

D'autres procédés, testés afin d'éliminer les HAP, consistent à utiliser des bactéries telles que *Pseudomonas*, *Sphingomonas* ou *Rhodococcus* qui ont la capacité d'absorber des HAP de 2 et 3 cycles tels que le naphtalène et le phénanthrène. Certains HAP peuvent être dégradés par oxydation en présence de champignons lignolytiques tels que le *Phanerochaete chrysosporium* ou bien des champignons microscopiques tels que le *Cunninghamella elegans*. Cependant, ces procédés conduisent le plus souvent à la dégradation complète des molécules aromatiques. L'application de ces procédés aux extraits aromatiques, outre le coût prohibitif de ces bactéries ou champignons et leur mise en oeuvre délicate en unités industrielles, va conduire à l'obtention de composés de propriétés très différentes à celles de l'extrait aromatique actuellement incorporé dans les pneumatiques et les caoutchoucs industriels.

Un autre moyen est d'opérer une ouverture partielle des cycles aromatiques en les oxydant de façon contrôlée afin de former des dérivés époxydés et/ou hydroxylés, par exemple des dérivés aldéhydes, des quinones, des acides et/ou des alcools ou encore des composés mixtes dont la toxicité est moindre au regard de l'environnement.

C'est ainsi que la Demanderesse, dans son brevet FR2888246, décrit l'oxydation des composés polycycliques aromatiques présents dans des coupes de distillation atmosphérique du pétrole brut, notamment des coupes essence, kérosène et gazole en présence d'un oxydant et d'hémoprotéine immobilisée ou non sur un support. Comme les extraits aromatiques, ces coupes contiennent des composés polycycliques aromatiques PCA, mais parmi ceux-ci, ce procédé d'oxydation est dirigé vers la dégradation de composés réfractaires aux traitements classiques d'hydrogénation, en particulier les dérivés benzothiophéniques et pyrroliques. Du fait de la fluidité des coupes et du faible taux de composés aromatiques réfractaires, ce procédé se fait dans un milieu essentiellement aqueux, indifféremment dans un solvant réactif ou non réactif, par mise en contact d'un agent oxydant avec la dite coupe à traiter en présence d'une hémoprotéine immobilisée ou supportée sur des particules solides divisées.

Le brevet US 6,461,859 décrit un procédé d'oxydation des composés soufrés de coupes pétrolières à l'aide d'une hémoprotéine, dans lequel les PCA soufrés sont oxydés dans une première étape puis éliminés du milieu dans une seconde étape de distillation.

Dans le cadre de la présente invention, la demanderesse vise la dégradation des HAP présents dans un extrait aromatique, dont on veut conserver les propriétés et qui sont beaucoup plus visqueux et donc plus difficiles à traiter que les coupes gazole, essence et kérosène. La viscosité des extraits aromatiques est telle qu'on ne peut pas transposer à l'identique le procédé utilisé dans le brevet FR 2888246 directement aux extraits aromatiques. Notamment, la mise en contact de l'hémoprotéine avec l'extrait aromatique est soumise à de nombreux écueils tels que l'emploi d'un solvant visant à baisser la viscosité de l'extrait aromatique qui sera essentiellement organique et qui ne devra pas inhiber ou ralentir la réaction d'oxydation.

La présente invention vise donc un procédé permettant de dégrader in situ les HAP sans modifier les propriétés mécaniques et physiques de l'extrait aromatique. En outre, elle vise la mise en oeuvre d'un procédé efficace à des coûts relativement faibles. Cette dégradation des HAP devra pouvoir se faire aussi bien en continu, en discontinu ou en semi continu selon la quantité d'extrait aromatique à traiter.

La présente invention comme définie dans la revendication 1 a donc pour objet un procédé de réduction de la teneur en hydrocarbures aromatiques polycycliques ou HAP dans les extraits aromatiques qui consiste à oxyder les HAP en présence d'une hémoprotéine par un agent oxydant caractérisé en ce que l'extrait aromatique est mis en contact avec l'agent oxydant dans un solvant organique non réactif, avant d'être mis en contact avec l'hémoprotéine immobilisée ou supportée.

Un premier avantage de ce procédé réside dans le fait que le mélange extrait aromatique/agent oxydant est rendu homogène avant d'être mis en contact avec le catalyseur de la réaction, ici l'hémoprotéine supportée ou non. Cette mise en contact est facilitée par l'abaissement de la viscosité du mélange au moyen d'un solvant approprié facilitant l'homogénéisation du mélange.

Un autre avantage est que seuls les composés HAP sont réduits, ceux-ci s'oxydant en premier, et que la totalité des caractéristiques de ces extraits restent inchangées.

Dans un mode particulier de la présente invention, le mélange extrait aromatique, solvant et agent oxydant est préférablement homogénéisé avant sa mise en contact avec l'hémoprotéine pour favoriser les contacts et donc l'efficacité du procédé.

La température de mise en contact du mélange extrait aromatique, solvant et agent oxydant avec l'hémoprotéine varie de 15 à 80°C, de préférence de 25 à 45°C.

Selon un mode complémentaire, le procédé comprend une étape ultime de séparation du solvant organique de l'extrait aromatique traité, c'est-à-dire oxydé : le solvant organique ainsi récupéré sera avantageusement recyclé, une étape préalable d'épuration n'étant pas exclue.

Le solvant organique est non réactif, c'est-à-dire qu'il ne réagit pas avec le mélange extrait aromatique, agent oxydant et hémoprotéine et ne forme pas de liaisons chimiques, en particulier covalentes avec les constituants du mélange. Le solvant organique est choisi dans le groupe constitué par les dialkyles cétones, les carboxylates d'alkyls, les N-alkylpyrrolidones et le diméthylsulfoxyde ou DMSO susceptibles de diluer les extraits aromatiques. Préférentiellement, ce solvant est choisi parmi la méthyléthylcétone, l'acétone, l'éthanoate d'éthyle, le méthylisobutylcétone, l'acétate d'éthyle, le N-méthylpyrrolidone (NMP). Ce choix est particulièrement important car le solvant ne doit pas dénaturer l'hémoprotéine utilisée ou inhiber la réaction d'oxydation.

Dans ce mode de réalisation de l'invention, l'agent oxydant est choisi parmi les composés oxydants solubles en milieu organiques. Parmi ceux-ci, l'oxygène moléculaire (O₂), l'air, l'ozone (O₃), le peroxyde d'hydrogène naissant (H₂O₂), les peroxydes organiques ou minéraux, les hydroperoxydes alkylés, les hydroperoxydes d'aryles et les peracides sont préférés.

L'invention est principalement appliquée aux extraits aromatiques comprenant plus de 10% de composés aromatiques polycycliques ou PCA, et de préférence plus de 20%.

En outre, ces extraits aromatiques comprennent moins de 70% en poids d'un mélange de composés naphténiques et paraffiniques.

En général, ces extraits aromatiques contiennent moins de 500 ppm de HAP, de préférence entre 5 et 300 ppm.

Les extraits aromatiques, objet du procédé selon l'invention, appartiennent préférentiellement au groupe constitué par les extraits aromatiques de distillats sous vide tels que le DAE (distillate aromatic extract en anglais), le MES et/ou d'extraits aromatiques résiduels ou RAE ou encore tout extrait résultant d'une extraction de ces extraits aromatiques tels que le TDAE et/ou le TRAE.

Dans un mode préféré de l'invention, le mélange extrait aromatique, solvant et agent oxydant est choisi dans un rapport de concentration respectif (en poids) de ces composés variant de 40-10/90-60/0.001-2, et de préférence variant de 30-20/80-70/0.1-1. De façon plus précise le procédé selon l'invention comprend les étapes suivantes dans l'ordre précisé:
i) une étape de dissolution de 10-40% en poids d'extrait aromatique dans un solvant organique,
ii) la mise en contact de l'extrait dilué au solvant avec l'agent oxydant puis l'homogénéisation du mélange,
iii) la mise en contact par balayage ou immersion de l'hémoprotéine immobilisée avec le mélange homogénéisé de l'étape ii),
iv) la récupération puis la séparation de l'extrait traité d'avec le solvant, et éventuellement,
v) le recyclage de solvant à l'étape i), de préférence après purification de ce dernier.

Pour mettre en oeuvre l'invention, l'hémoprotéine immobilisée ou supportée est choisie parmi les hémoglobines et les myoglobines.

On a constaté également que le mode d'immobilisation de l'hémoprotéine est important pour être efficace. Ainsi dans le cadre de la présente invention, l'hémoprotéine est immobilisée sur ou dans des particules solides minérales finement divisées ayant une taille moyenne, déterminée par granulométrie laser, comprise entre 5 nm et 5 mm. De préférence, ces particules ont une taille comprise entre 10 nm et 2 mm. Elles sont choisies parmi les matériaux cristallins, amorphes ou composites à base d'oxydes alcalins et/ou alcalino-terreux, de préférence parmi les matériaux comprenant de l'alumine, de la silice, de la zircone, de l'oxyde de titane ou de tout matériau composite comprenant au moins un de ces matériaux.

Pour immobiliser l'hémoprotéine, celle-ci est absorbée à la surface des particules solides et/ou dans les pores de celles-ci dans un rapport variant de 1 à 2000 mg d'hémoprotéine par g de particules minérales.

Le mode d'immobilisation de l'hémoprotéine sur ces particules solides peut se faire de deux façons ; soit par balayage d'une colonne remplie de ces particules solides par une solution aqueuse d'hémoprotéine, par exemple variant de 5 à 100g/l d'hémoprotéine, soit par immersion d'une hémoprotéine dans un mélange eau/acétone, par exemple dans un rapport relatif **1/3** en volume, le solide étant séché avant utilisation.

Parmi les hémoprotéines envisagées, l'hémoprotéine, préférentiellement choisie parmi les hémoglobines de boeuf et/ou de cochon, est immobilisée sur de la silice divisée de taille particulaire variant de 5 nm et 5 mm, à une teneur variant de 10 à 200mg/g de silice.

On pourra se référer aussi à la demande de la demanderesse précitée pour plus de détails relativement à l'hémoprotéine, son procédé de préparation, le support, le procédé d'immobilisation, etc.

L'invention va être décrite ci-après au regard des deux figures et des exemples donnés ci-après.

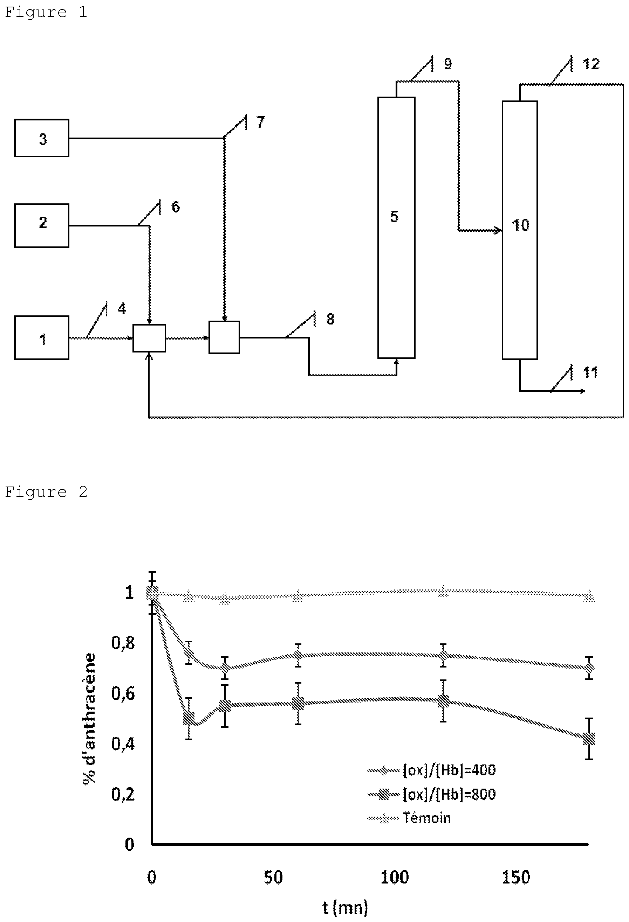

La figure 1 représente le schéma du procédé selon l'invention dans une mise en oeuvre en continu du procédé selon l'invention.
   Sur la figure 1 sont représentées trois capacités de stockage de l'extrait aromatique (1), du solvant (2) et de l'oxydant (3). La conduite (4) envoie l'extrait aromatique au réacteur (5) contenant l'hémoprotéine supportée sur particules solides. Les conduites (6) et (7) ramènent dans la conduite (4) successivement le solvant permettant de diluer l'extrait aromatique, puis l'oxydant, l'ensemble pouvant être homogénéisé dans un mode non représenté. Le mélange homogénéisé est introduit dans le réacteur (5) via la conduite (8). L'extrait oxydé en mélange dans le solvant est évacué via la conduite (9) vers une capacité de séparation (10), par exemple une colonne de distillation, l'extrait oxydé étant récupéré via la conduite (11) et le solvant par la conduite (12). Cette conduite (12) permet de recycler le solvant via éventuellement un traitement de purification non représenté.
La figure 2 représente le % de disparition de l'anthracène avec du furfural comme solvant, en fonction du temps exprimé en minutes par rapport à un témoin ne contenant pas d'agent oxydant et pour deux rapports en poids oxydant/ hémoprotéine, respectivement **de 400 et de 800**.
La figure 3 représente le % de disparition de l'anthracène avec de la méthyléthylcétone comme solvant, en fonction du temps exprimé en minutes par rapport à un témoin ne contenant pas d'agent oxydant et pour deux rapports en poids oxydant/ hémoprotéine, respectivement **de 400 et de 800**.
La figure 4 représente le % de disparition de l'anthracène avec de la méthyléthylcétone comme solvant, en fonction du temps exprimé en minutes.

Les exemples donnés ci-après visent à démontrer l'intérêt de l'invention sans en limiter la portée.

### EXEMPLE 1

L'objet de cet exemple est de décrire la préparation d'une hémoprotéine immobilisée sur des particules solides divisées selon deux modes de préparation, le mode batch pour lequel l'hémoprotéine est absorbée dans les particules solides et le mode continu consistant à balayer un lit de particules solides divisées par une solution d'hémoprotéine.

Le mode en batch donne une hémoprotéine X immobilisée sur silice, ci-après référencée hémoprotéine X.

Dans un récipient adapté à une centrifugeuse, on ajoute 1 g de silice Aerosil^{®} 200 (Degussa), 200 mg d'hémoglobine bovine (3,1 µmol) et 10 mL de solution tampon phosphate (pH 6, 50 mM). Le mélange est agité à 0 °C pendant 5 mn, puis 30 mL d'acétone sont ajoutés goutte-à-goutte sur une période de 10 min. On poursuit l'agitation du mélange pendant 30 mn à 0°C, puis on centrifuge à 3000 tours/mn pendant 10 min. Le résidu solide collecté dans le fond de l'éprouvette est lavé avec 10 mL de tampon phosphate (pH 6) puis centrifugé à 3000 tours/mn pendant 10 mn, 3 fois de suite. Le solide résultant est ensuite séché sous vide en présence de P₂O₅. L'hémoprotéine X contient 133 mg d'hémoglobine par gramme de silice.

Le mode continu donne une hémoprotéine Y immobilisée sur silice, ci-après référencée hémoprotéine Y.

Une solution aqueuse contenant 10 g/l d'hémoglobine bovine est mise en circulation à raison de 1 ml/mn à travers une colonne HPLC préparative en acier inox 316 de 10 mm de diamètre interne et 250 mm de long. Cette colonne est remplie avec 7.9 g de silice Silicagel^{®}100 (Merck). La quantité d'hémoglobine fixée sur la silice est mesurée par différence de concentration en hémoglobine de la solution entre l'entrée et la sortie de la colonne par mesure spectrométrique UV-Vis à λ=404nm. Lorsque la quantité d'hémoglobine immobilisée sur la silice est suffisante, on fait circuler à travers la colonne une solution aqueuse additionnée d'une concentration croissante en acétone, de 100% d'eau jusqu'à 1 à 5% d'eau, pour entraîner l'excédent d'hémoglobine non absorbée et sécher l'hémoprotéine Y. Par cette méthode, la quantité d'hémoprotéine adsorbée sur la silice est d'environ 90 mg/g de silice.

### EXEMPLE 2

Dans le présent exemple, on compare l'effet de la nature du solvant sur la dégradation de l'anthracène, molécule modèle pour les HAP.

On a comparé la dégradation de l'anthracène en présence d'un agent oxydant, soit en solution dans du furfural soit en solution avec de la méthyléthylcétone (ou MEK - en anglais methylethylketon) dans les mêmes conditions réactionnelles. Deux solutions mères contenant environ 100 ppm d'anthracène ont donc été préparées respectivement dans du furfural et de la MEK. Ces solutions on été utilisées pour préparer des solutions étalons pour la mesure de l'anthracène dans les solutions dégradées au moyen de la HPLC-UV (λ=251,1 nm). Dans des ballons de 100 ml placés sous agitation magnétique, on introduit 16 ml de solvant (furfural ou MEK), puis 4 ml de la solution mère d'anthracène (soit une concentration en anthracène de 20 ppm dans le mélange correspondant au niveau de concentrations des HAP dans une solution industrielle) et enfin, 800 mg d'hémoprotéine supportée. Après homogénéisation du mélange ainsi obtenu, on ajoute entre 20 et 40 µl d'une solution du tertbutyl peroxyde à 70 % dans l'eau, puis le chronomètre est déclenché. Des prélèvements réguliers sont effectués dans chacun des deux mélange contenant du furfural ou du MEK : ils sont dilués dans l'acétonirile puis analysés par HPLC.

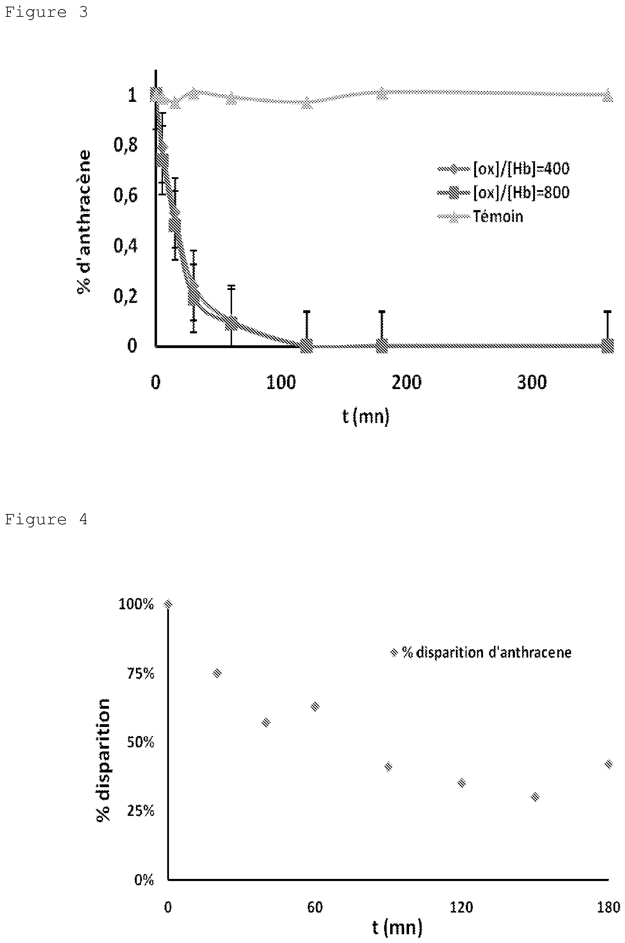

Dans les figures 2 et 3, sont représentées la dégradation de l'anthracène se traduisant par une baisse de la concentration en anthracène, en présence d'un oxydant respectivement en présence de furfural (figure 2) et de MEK (figure 3) comparativement à un échantillon témoin sans agent oxydant.

On notera que le furfural inhibe la réaction de dégradation de l'anthracène pour les deux rapports oxydant/hémoglobine ; le furfural donne donc des résultats inférieures à la MEK.

### EXEMPLE 3

Dans le présent exemple, on décrit la dégradation de l'anthracène en continu en présence d'une hémoprotéine Y décrite dans l'exemple 1.

La colonne, remplie d'hémoprotéine Y est alimentée en continu à un débit de 1 ml/mn, par une solution de MEK contenant 150 ppm d'anthracène (ANT) et de tertbutylperoxyde (tBuOH), le rapport molaire [tBuOH]/[ANT] étant fixé à 300. En comparant les teneurs en anthracène mesurées entre l'entrée et la sortie de la colonne par GC-MS (couplage chromatographie en phase gazeuse /spectromètrie de masse), on détermine la quantité d'anthracène dégradé. Le graphe correspondant à la disparition de l'anthracène en fonction du temps est donné en figure 4.

### EXEMPLE 4

Dans le présent exemple, on décrit la dégradation de des HAP d'un extrait aromatique selon un mode continu en présence d'une hémoprotéine Y comme décrit dans l'exemple 3.

L'extrait aromatique est un extrait de type DAE contenant 15-25 % de PCA dont 1-0.01% de HAP (TOTAL EXAROL 41).

On mesure comme précédemment la teneur de plusieurs HAP contenus dans le DAE entre l'entrée et la sortie de la colonne. Les résultats sont donnés dans le tableau I ci-après.

**TABLEAU I**

| | **Concentration HAP Avant colonne** | **Concentration HAP Après colonne** | **Dégradation** |
|---|---|---|---|
| | **(ppm)** | **(ppm)** | **(%)** |
| **Benzo (a) anthracene** | 6,5 | 5,4 | 18% |
| **Chrysène** | 26,4 | 23,5 | 11% |
| **Benzo (b) fluoranthène** | 16,3 | 12,8 | 21% |
| **Benzo (k) fluoranthène** | 5,5 | 4,2 | 23% |
| **Benzo (a) pyrène** | 18,5 | 13,3 | 28% |
| **Dibenzo (a,h) anthracène** | 17,4 | 11,5 | 34% |
| **Benzo (b+j) fluoranthène** | 16,3 | 12,8 | 21% |
| **Benzo (e) pyrène** | 64,6 | 42,7 | 34% |
| **TOTAL** | 171,5 | 126,2 | 26% |

Le procédé selon l'invention permet de dégrader les HAP mentionnés dans la Directive européenne à plus de 20% dans la majorité des cas.

## Revendications

1. Procédé de réduction de la teneur en hydrocarbures aromatiques polycycliques ou HAP dans les extraits aromatiques qui consiste à oxyder les HAP en présence d'une hémoprotéine par un agent oxydant **caractérisé en ce que** l'extrait aromatique est mis en contact avec l'agent oxydant dans un solvant organique non réactif choisi dans le groupe constitué par les dialkyles cétones, les carboxylates d'alkyles, les N-alkylpyrrolidones et le diméthylsulfoxyde (DMSO), puis est mis en contact avec l'hémoprotéine immobilisée ou supportée.

2. Procédé selon la revendication 1 **caractérisé en ce que** le procédé comprend une étape d'homogénéisation du mélange extrait aromatique, solvant et agent oxydant avant la mise en contact avec l'hémoprotéine.

3. Procédé selon l'une des revendications précédentes **caractérisé en ce que** la température de mise en contact du mélange extrait aromatique, solvant et agent oxydant avec l'hémoprotéine varie de 15 à 80°C, de préférence de 25 à 45°C.

4. Procédé selon l'une des revendications précédentes, **caractérisé en ce que** le procédé comprend une étape ultime de séparation de l'extrait aromatique traité du solvant organique qui est recyclé.

5. Procédé selon l'une des revendications précédentes **caractérisé en ce que** le solvant organique est choisi dans le groupe constitué par la méthyléthylcétone, l'acétone, l'éthanoate d'éthyle, le méthylisobutylcétone, l'acétate d'éthyle, le N-méthylpyrrolidone (NMP).

6. Procédé selon l'une des revendications précédentes **caractérisé en ce que** l'agent oxydant est choisi parmi les composés oxydants solubles en milieu organiques.

7. Procédé selon l'une des revendications précédentes **caractérisé en ce que** l'agent oxydant est choisi parmi l'oxygène moléculaire (O₂), l'air, l'ozone (O₃), le peroxyde d'hydrogène naissant (H₂O₂), les peroxydes organiques ou minéraux, les hydroperoxydes alkylés, les hydroperoxydes d'aryles et les peracides.

8. Procédé selon l'une des revendications précédentes **caractérisé en ce que** les extraits aromatiques comprennent plus de 10% de composés aromatiques polycycliques ou PCA et de préférence plus de 20%.

9. Procédé selon l'une des revendications précédentes **caractérisé en ce que** les extraits aromatiques comprennent moins de 70% en poids d'un mélange de composés naphténiques et paraffiniques.

10. Procédé selon l'une des revendications précédentes **caractérisé en ce que** les extraits aromatiques sont choisis dans le groupe constitué par les extraits aromatiques de distillats sous vide, les solvats d'extraction modérée (mild extract solvates « MES ») et/ou les extraits aromatiques résiduels (residual aromatic extracts « RAE ») ou encore tout extrait résultant d'une extraction de ces extraits aromatiques tels que l'extrait aromatique de distillat traité (treated distillate aromatic extract « TDAE ») et/ou l'extrait aromatique résiduel traité par double extraction (treated residual aromatic extract « TRAE »).

11. Procédé selon l'une des revendications précédentes **caractérisé en ce que** le mélange extrait aromatique, solvant et agent oxydant correspond à un rapport en poids respectif de ces composés variant de 40-10/90-60/0.001-2, de préférence de 30-20/80-70/0.1-1.

12. Procédé selon l'une des revendications précédentes **caractérisé en ce que** l'extrait aromatique est dilué dans le solvant organique avant d'être mélangé avec l'agent oxydant, puis homogénéisé.

13. Procédé selon l'une des revendications précédentes **caractérisé en ce qu'**il comprend :
i) une étape de dissolution de 10 à 40% en poids d'extrait aromatique dans un solvant organique,
ii) la mise en contact de l'extrait dilué au solvant avec l'agent oxydant puis l'homogénéisation du mélange,
iii) la mise en contact par balayage ou immersion de l'hémoprotéine immobilisée avec le mélange homogénéisé de l'étape ii),
iv) la récupération puis la séparation de l'extrait traité d'avec le solvant, et éventuellement,
v) le recycle de solvant à l'étape i) éventuellement après purification de ce dernier.

14. Procédé selon l'une des revendications précédentes **caractérisé en ce que** l'hémoprotéine immobilisée ou supportée est choisie parmi les hémoglobines et les myoglobines.

15. Procédé selon l'une des revendications précédentes **caractérisé en ce que** l'hémoprotéine est immobilisée sur ou dans des particules solides minérales finement divisées ayant une taille moyenne, déterminée par granulométrie laser, comprise entre 5 nm et 5 mm, de préférence comprise entre 10 nm et 2 mm, ces particules étant choisies dans le groupe des matériaux cristallins, amorphes ou composites à base d'oxydes alcalins et/ou alcalino-terreux, de préférence parmi les matériaux comprenant de l'alumine, de la silice, de la zircone, de l'oxyde de titane ou de tout matériau composite comprenant au moins un de ces matériaux.

16. Procédé selon l'une des revendications précédentes **caractérisé en ce que** l'hémoprotéine est absorbée à la surface des particules solides et/ou dans les pores de celles-ci dans un rapport variant de 1 à 2000 mg d'hémoprotéine par gramme de particules minérales.

17. Procédé selon l'une des revendications précédentes **caractérisé en ce que** l'hémoprotéine est une hémoglobine immobilisée sur les particules solides et/ou dans les pores de celles-ci dans un rapport variant de 1 à 2000 mg d'hémoprotéine par gramme de particules minérales.

## Patentansprüche

1. Verfahren zur Verringerung des Gehalts an polyzyklischen aromatischen Kohlenwasserstoffen oder PAK in aromatischen Extrakten, das aus dem Oxidieren der PAK in Gegenwart eines Hämoproteins mit einem Oxidationsmittel besteht, **dadurch gekennzeichnet, dass** der aromatische Extrakt mit dem Oxidationsmittel in Kontakt gebracht wird in einem nicht reaktiven organischen Lösemittel, ausgewählt aus der Gruppe bestehend aus Dialkylketonen, Alkylcarboxylaten, N-Alkylpyrrolidonen und Dimethylsulfoxid (DMSO), und daraufhin mit dem immobilisierten oder geträgerten Hämoprotein in Kontakt gebracht wird.

2. Verfahren nach Anspruch 1, **dadurch gekennzeichnet, dass** das Verfahren einen Schritt der Homogenisierung des Gemischs aus aromatischem Extrakt, Lösemittel und Oxidationsmittel vor dem in Kontakt bringen mit dem Hämoprotein umfasst.

3. Verfahren nach einem der vorherigen Ansprüche, **dadurch gekennzeichnet, dass** die Kontakttemperatur des Gemischs aus aromatischem Extrakt, Lösemittel und Oxidationsmittel mit dem Hämoprotein im Bereich von 15 bis 80 °C liegt, vorzugsweise von 25 bis 45 °C.

4. Verfahren nach einem der vorherigen Ansprüche, **dadurch gekennzeichnet, dass** das Verfahren einen letzten Schritt der Abtrennung des behandelten aromatischen Extrakts von dem organischen Lösemittel umfasst, das rückgeführt wird.

5. Verfahren nach einem der vorherigen Ansprüche, **dadurch gekennzeichnet, dass** das organische Lösemittel ausgewählt wird aus der Gruppe bestehend aus Methylethylketon, Aceton, Ethylethanoat, Methylisobutylketon, Ethylacetat, N-Methylpyrrolidon (NMP).

6. Verfahren nach einem der vorherigen Ansprüche, **dadurch gekennzeichnet, dass** das Oxidationsmittel ausgewählt wird aus oxidierenden Verbindungen, die in einem organischen Medium löslich sind.

7. Verfahren nach einem der vorherigen Ansprüche, **dadurch gekennzeichnet, dass** das Oxidationsmittel ausgewählt wird aus molekularem Sauerstoff (O₂), Luft, Ozon (O₃), naszierendem Wasserstoffperoxid (H₂O₂), organischen oder anorganischen Peroxiden, Alkylhydroperoxiden, Arylhydroperoxiden und Persäuren.

8. Verfahren nach einem der vorherigen Ansprüche, **dadurch gekennzeichnet, dass** die aromatischen Extrakte mehr als 10%, und vorzugsweise mehr als 20%, an polyzyklischen aromatischen Verbindungen oder PCA enthalten.

9. Verfahren nach einem der vorherigen Ansprüche, **dadurch gekennzeichnet, dass** die aromatischen Extrakte weniger als 70 Gewichts-% eines Gemischs aus naphthenischen und paraffinischen Verbindungen umfassen.

10. Verfahren nach einem der vorherigen Ansprüche, **dadurch gekennzeichnet, dass** die aromatische Extrakte ausgewählt werden aus der Gruppe bestehend aus aromatischen Extrakten von Vakuumdestillaten, aus milden Extraktionssolvaten (mild extract solvaten "MES") und/oder aromatischen Extraktrückständen (residual aromatic extracts "RAE") oder auch aus sämtlichen Extrakten, die aus der Extraktion dieser aromatischen Extrakte resultierten, wie etwa aromatischem Extrakt aus behandeltem Destillat (treated distillate aromatic extract "TDAE") und/oder wie restlichem aromatischem Extrakt behandelt durch Doppelextraktion (treated residual aromatic extract "TRAE").

11. Verfahren nach einem der vorherigen Ansprüche, **dadurch gekennzeichnet, dass** das Gemisch aus aromatischem Extrakt, Lösemittel und Oxidationsmittel einem Gewichtsverhältnis in Bezug auf das jeweilige Gewicht dieser Verbindungen im Bereich von 40-10/90-60/0,001-2, vorzugsweise von 30-20/80-70/0,1-1 entspricht.

12. Verfahren nach einem der vorherigen Ansprüche, **dadurch gekennzeichnet, dass** der aromatische Extrakt in dem organischen Lösemittel verdünnt wird, bevor er mit dem Oxidationsmittel vermischt wird, und daraufhin homogenisiert wird.

13. Verfahren nach einem der vorherigen Ansprüche, **dadurch gekennzeichnet, dass** es Folgendes umfasst:
i) einen Schritt des Lösens von 10 bis 40 Gewichts-% aromatischen Extrakts in einem organischen Lösemittel,
ii) das in Kontakt bringen des verdünnten Extrakts mit dem Oxidationsmittel, und daraufhin Homogenisieren des Gemischs,
iii) das in Kontakt bringen durch Spülen oder Eintauchen des immobilisierten Hämoproteins mit dem homogenisierten Gemisch aus Schritt ii),
iv) die Rückgewinnung und daraufhin Trennung des mit dem Lösemittel behandelten Extrakts, und gegebenenfalls,
v) das Rückführen des Lösemittels zu Schritt i), gegebenenfalls nach einer Reinigung desselben.

14. Verfahren nach einem der vorherigen Ansprüche, **dadurch gekennzeichnet, dass** das immobilisierte oder geträgerte Hämoprotein ausgewählt wird aus Hämoglobinen und Myoglobinen.

15. Verfahren nach einem der vorherigen Ansprüche, **dadurch gekennzeichnet, dass** das Hämoprotein auf oder in feinteiligen anorganischen Feststoffteilchen immobilisiert wird, mit einer mittleren Größe, bestimmt durch Lasergranulometrie, zwischen 5 nm und 5 mm, vorzugsweise zwischen 10 nm und 2 mm, wobei diese Teilchen ausgewählt werden aus der Gruppe amorpher kristalliner Materialien, oder Verbundmaterialien auf Basis von Alkali- und/oder Erdalkalioxiden, vorzugsweise aus Materialien, die Aluminiumoxid, Siliciumoxid, Zirkoniumoxid, Titanoxid umfassen oder sämtlichen Verbundmaterialien, die mindestens eines dieser Materialien umfassen.

16. Verfahren nach einem der vorherigen Ansprüche, **dadurch gekennzeichnet, dass** das Hämoprotein an der Oberfläche der festen Teilchen und/oder in den Poren derselben absorbiert werden in einem Verhältnis im Bereich von 1 bis 2000 mg Hämoprotein pro Gramm Mineralteilchen.

17. Verfahren nach einem der vorherigen Ansprüche, **dadurch gekennzeichnet, dass** das Hämoprotein ein Hämoglobin ist, das auf den festen Teilchen und/oder in den Poren derselben in einem Verhältnis im Bereich von 1 bis 2000 mg Hämoprotein pro Gramm Mineralpartikel immobilisiert ist.

## Claims

1. Method for reducing the content of polycyclic aromatic hydrocarbons or PAHs in aromatic extracts, which consists in oxidizing the PAHs in the presence of a haemoprotein using an oxidizing compound **characterized in that** the aromatic extract is brought into contact with the oxidizing agent in a non-reactive organic solvent selected from the group consisting of dialkyl ketones, alkyl carboxylates, N-alkylpyrrolidones and dimethylsulphoxide (DMSO) and is then brought into contact with the immobilized or supported haemoprotein.

2. Method according to claim 1 **characterized in that** the method comprises a step of homogenization of the aromatic extract, solvent and oxidizing agent mixture before it is brought into contact with the haemoprotein.

3. Method according to one of the previous claims **characterized in that** the temperature at which the aromatic extract, solvent and oxidizing agent mixture is brought into contact with the haemoprotein varies from 15 to 80°C, preferably from 25 to 45°C.

4. Method according to one of the previous claims, **characterized in that** the method comprises a final step of separation of the treated aromatic extract from the organic solvent which is recycled.

5. Method according to one of the previous claims **characterized in that** the organic solvent is chosen from the group constituted by methyl ethyl ketone, acetone, ethyl ethanoate, methyl isobutyl ketone, ethyl acetate, N-methylpyrrolidone (NMP).

6. Method according to one of the previous claims **characterized in that** the oxidizing agent is chosen from oxidizing compounds soluble in organic medium.

7. Method according to one of the previous claims **characterized in that** the oxidizing agent is chosen from molecular oxygen (O₂), air, ozone (O₃), nascent hydrogen peroxide (H₂O₂), organic or mineral peroxides, alkylated hydroperoxides, aryl hydroperoxides and peracids.

8. Method according to one of the previous claims **characterized in that** the aromatic extracts comprise more than 10% of polycyclic aromatic compounds or PCA and preferably more than 20%.

9. Method according to one of the previous claims **characterized in that** the aromatic extracts comprise less than 70% by weight of a mixture of naphthenic and paraffinic compounds.

10. Method according to one of the previous claims **characterized in that** the aromatic extracts are chosen from the group constituted by the aromatic extracts of vacuum distillates, mild extract solvates "MES" and/or residual aromatic extracts "RAEs" or also any extract resulting from an extraction of these aromatic extracts such as the treated distillate aromatic extract "TDAE" and/or the treated residual aromatic extract "TRAE".

11. Method according to one of the previous claims **characterized in that** the aromatic extract, solvent and oxidizing agent mixture corresponds to a respective weight ratio of these compounds varying from 40-10/90-60/0.001-2, preferably from 30-20/80-70/0.1-1.

12. Method according to one of the previous claims **characterized in that** the aromatic extract is diluted in the organic solvent before being mixed with the oxidizing agent, then homogenized.

13. Method according to one of the previous claims **characterized in that** it comprises:
i) a step of dissolution of 10 to 40% by weight of aromatic extract in an organic solvent,
ii) bringing the extract diluted in the solvent into contact with the oxidizing agent, then homogenizing the mixture,
iii) bringing the immobilized haemoprotein into contact with the homogenized mixture of step ii) by flushing or immersion,
iv) recovering, then separating the treated extract from the solvent and, optionally,
v) recycling the solvent at step i), optionally after purification of the latter.

14. Method according to one of the previous claims **characterized in that** the immobilized or supported haemoprotein is chosen from the haemoglobins and the myoglobins.

15. Method according to one of the previous claims **characterized in that** the haemoprotein is immobilized on or in finely divided solid mineral particles having an average size, determined by laser granulometry, comprised between 5 nm and 5 mm, preferably comprised between 10 nm and 2 mm, these particles being chosen from the group of the crystalline, amorphous or composite materials based on alkaline or alkaline-earth oxides, preferably from the materials comprising alumina, silica, zirconia, titanium oxide or any composite material comprising at least one of these materials.

16. Method according to one of the previous claims **characterized in that** the haemoprotein is absorbed on the surface of the solid particles and/or in the pores thereof in a ratio varying from 1 to 2000 mg of haemoprotein per g of mineral particles.

17. Method according to one of the previous claims **characterized in that** the haemoprotein is a haemoglobin immobilized on the solid particles and/or in the pores thereof in a ratio varying from 1 to 2000 mg of haemoprotein per g of mineral particles.
